# EUROPEAN PATENT SPECIFICATION

(11) **EP 4 533 945 B1**
(45) Date of publication and mention of the grant of the patent: **02.12.2026**
(21) Application number: 25158242.5
(22) Date of filing: 07.09.2016
(51) Int. Cl.: A01N 1/143

(54) **AORTIC CANNULA FOR EX VIVO ORGAN CARE SYSTEM**
AORTENKANÜLE FÜR EX-VIVO-ORGANPFLEGESYSTEM
CANULE AORTIQUE POUR SYSTÈME DE SOINS D'ORGANES EX VIVO

(30) Priority: 09.09.2015 US 201562215825 P
(43) Date of publication of application: 09.04.2025
(62) Document number(s) of the earlier application(s) in accordance with Art. 76 EPC: 20206681.7 / 3 824 731; 16844964.3 / 3 347 084
(73) Proprietor: TransMedics, Inc., Andover, MA 01810 (US)
(72) Inventor: RITCHIE, Greg, Rawley, 01969 (US); LAMBERT II, Vincent, Salisbury, 01952 (US); BRINGHAM, Richard, North Andover, 01845 (US); SULLIVAN, John, Groton, 01450 (US); HASSANEIN, Waleed, Andover, 01845 (US)
(74) Representative: FRKelly

(56) References cited:
- WO-A1-03/026419
- WO-A1-2014/194349
- US-A1- 2012 277 681
- DIANA GARCÍA SÁEZ ET AL: "Evaluation of the Organ Care System in Heart Transplantation With an Adverse Donor/Recipient Profile", THE ANNALS OF THORACIC SURGERY, vol. 98, no. 6, 1 December 2014 (2014-12-01), United States, pages 2099 - 2106, XP055581439, ISSN: 0003-4975, DOI: 10.1016/j.athoracsur.2014.06.098

## Description

### TECHNICAL FIELD

The present invention relates generally to medical devices and, in particular, aortic cannulas for use in ex vivo organ care systems. Specifically the invention relates to aortic cannulas used to return perfusate to the heart or delivering perfusate from the heart while the organ is sustained ex vivo at physiologic or near-physiologic conditions.

### BACKGROUND

Current organ preservation techniques typically involve hypothermic storage of the organ in a chemical perfusate solution on ice. However, uses of conventional approaches results in injuries that increase as a function of the length of time an organ is maintained ex-vivo. These time restrictions limit the number of recipients who can be reached from a given donor site, thereby restricting the recipient pool for a harvested heart. Even within the few hour time limit, the heart may nevertheless be significantly damaged.

Effective preservation of an ex-vivo organ would also provide numerous other benefits. For instance, prolonged ex-vivo preservation would permit more careful monitoring and functional testing of the harvested organ. This would in turn allow earlier detection and potential repair of defects in the harvested organ, further reducing the likelihood of transplantation failure. The ability to perform simple repairs on the organ would also allow many organs with minor defects to be saved, whereas current transplantation techniques require them to be discarded. In addition, more effective matching between the organ and a particular recipient may be achieved, further reducing the likelihood of eventual organ rejection.

Improved ex-vivo organ care has been achieved through the use of an ex-vivo organ care system which maintains organs at physiologic or near-physiologic conditions. Not only does the system maintain the organ at physiologic temperatures, but in the case of the heart, the system maintains perfusate flow through the organ. In addition the system measures and monitors electric stimulation in the heart. The ex vivo organ care system where the heart sustained ex vivo at physiologic or near-physiologic conditions are described in Application Ser. No. 11/822495 entitled "Systems for monitoring and applying electrical currents in an organ perfusion system," U.S. Pat. No. 8,304,181 entitled "Method for ex-vivo organ care and for using lactate as an indication of donor organ status," and U.S. Pat. No. 8,409,846 entitled "Compositions, methods and devices for maintaining an organ".

To maintain physiologic or near-physiologic perfusate flow through the heart, the organ must interface with the system via the aorta. This interface is achieved via an aortic cannula. Current aortic cannula designs lead to organ slippage, difficulties in maintaining a liquid tight seal, and damage to the aorta. Often these designs rely solely upon a cable tie in contact with the aorta to tighten the aorta to the aortic cannula. Depending on the size of the aorta and the size of the aortic cannula, there is a risk of laceration due to the cable ties exerting too much tension on aortic tissue, or the risk of leakage if they do not exert sufficient tension. Thus, there exists a need for an aortic cannula that is easy for health care workers to deploy, creates a tight seal with the aorta, reduces aortic slipping, and causes minimal damage to the aorta.

US 2012 277681 A1 relates to a cannula including a first circumferential portion, a second circumferential portion, and a seal with a first clamping surface. The first circumferential portion and the second circumferential portion are configured to mutually cooperate to support a circumference of vasculature, and form a second clamping surface. The first clamping surface and the second clamping surface are configured to cooperate to secure an end of the vasculature.

In view of the foregoing, improved devices for attaching the aorta to the system and methods of use in ex vivo organ care systems are needed.

### SUMMARY

The invention is defined by appended claim 1. Advantageous features are provided in the dependent features. In accordance with the invention the aortic cannula for use with an *ex vivo* organ care system comprises: a cannula body which further comprises, a fitting adapted to connect to an organ care system, and an aorta interface to contact an aorta; and pivot comprising a pivot arm strap operably connected to a pivot mount, wherein the pivot mount allows the pivot arm strap to uniformly contact the aorta to hold the aorta on the aorta interface. In one embodiment, pivot arm is configured such that when the pivot arm is moved toward the cannula body by rotation around the pivot mount, the pivot arm strap moves away from the cannula body. In another embodiment of the aortic cannula the pivot arm and the pivot arm strap are parts of a single piece. In another embodiment, the aortic cannula comprises a spring which applies pressure to the pivot arm strap to hold the aorta on the aorta interface. In another embodiment of the aortic cannula a dowel pin communicates with the spring to allow the pivot arm to rotate around the dowel pin. In another embodiment of the aortic cannula the pivot arm further comprises a grip pad used to depress the top of the pivot arm. In another embodiment of the aortic cannula the grip pad is textured. In another embodiment of the aortic cannula the grip pad is removable. In another embodiment of the aortic cannula the pivot arm straps further comprise a loop and guide which retain a cable tie around the pivot arm strap. In another embodiment, the aortic cannula further comprises windows sized to normalize the compression exerted on the aorta by the cable tie such that the same amount of pressure will be exerted on the aorta regardless of the size of the pivot arm strap for a given cable tie tension. In another embodiment, the aortic cannula further comprises a connector used to reversibly secure the aortic cannula to an organ chamber. In another embodiment of the aortic cannula the connector is a threaded locking nut. In another embodiment of the aortic cannula the aorta interface is textured.

The present disclosure further includes a method of using an aortic cannula to place a heart in fluid communication with an organ care system the method comprising, selecting an aortic cannula sized to fit the aorta of the heart the aortic cannula comprising, a cannula body further comprising, a fitting adapted to connect to an organ care system, an aorta interface to contact an aorta, and a pivot arm strap operably connected to a pivot mount, wherein the pivot mount allows the pivot arm strap to uniformly contact the aorta to hold the aorta on the aorta interface, depressing the pivot arm such that it rotates around the dowel pin and the pivot arm strap moves away from the cannula body, placing the cannula in the aorta, releasing the pivot arm, tightening a cable tie around the pivot arm strap to hold the aorta in place, and inserting the tapered fitting into an organ care system. In one embodiment, the method further comprises the step of suturing surgical felt pledgets on the aorta before placing the aorta on the aortic cannula.

### BRIEF DESCRIPTION OF THE FIGURES

The following figures depict illustrative embodiments of the invention.
Fig. 1 illustrates a diagram depicting an aortic cannula in one embodiment.
Fig. 2a illustrates a side view of a cannula body in one embodiment.
Fig. 2b illustrates a side view of a cannula body and a spring pocket according to one embodiment.
Fig. 2c illustrates a side view of a cannula body in one embodiment.
Fig. 3a illustrates one embodiment of a pivot arm.
Fig. 3b illustrates a side view of a pivot arm and strap according to one embodiment.
Fig. 3c illustrates another view of a pivot arm and strap according to one embodiment.
Fig. 3d illustrates another view of a pivot arm and strap according to one embodiment.
Fig. 3e illustrates a top view of a pivot arm and strap according to one embodiment.
Fig. 4 illustrates a diagram showing the shape of a cannula body texture in one embodiment.
Fig. 5 illustrates a top view of a pivot mount according to one embodiment.
Fig. 6 illustrates a tip holder according to one embodiment.

### DETAILED DESCRIPTION

### Cannula Body

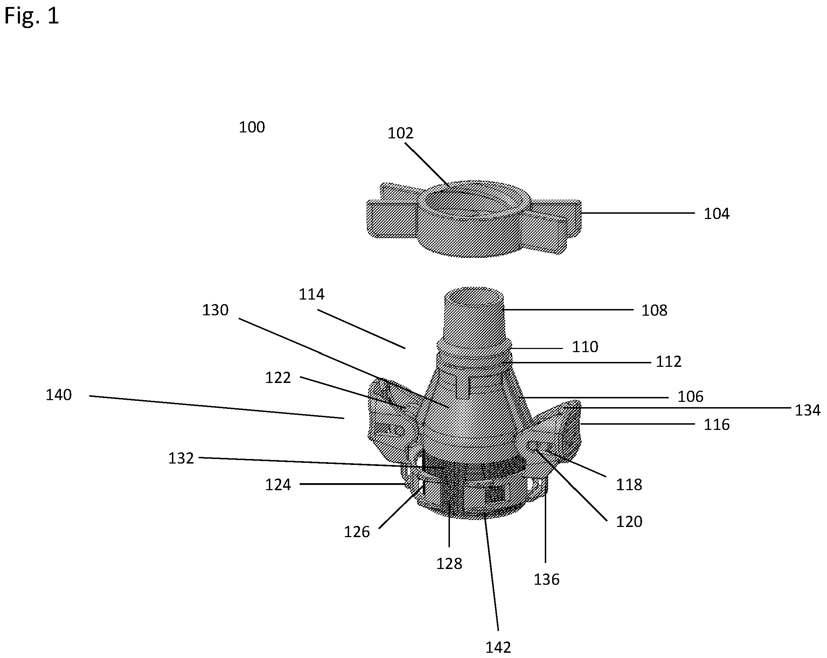

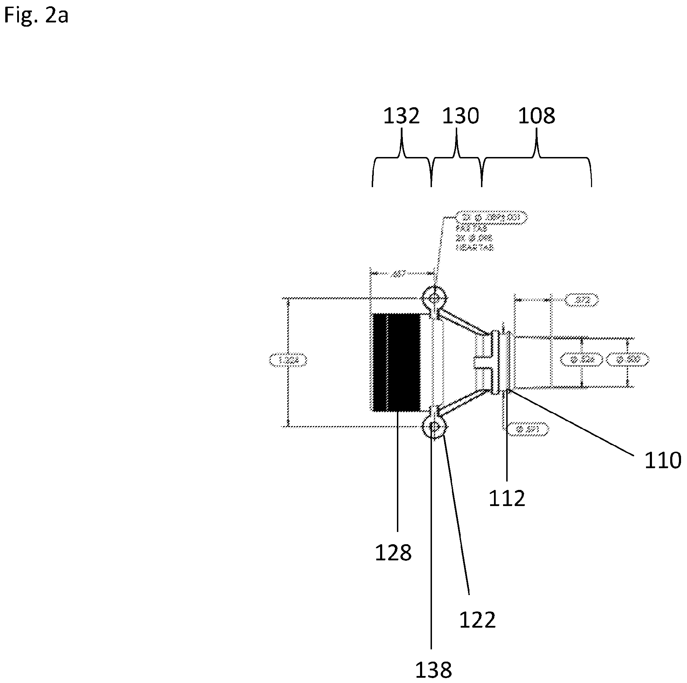

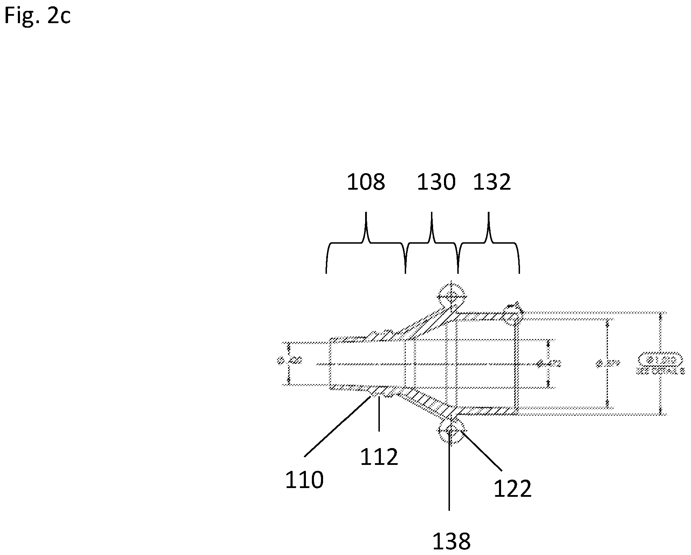

Figure 1 is a diagram depicting the aortic cannula 100 in one embodiment. The aortic cannula device 100 comprises a cannula body 114, a locking nut 102, and a pivot arm 140. The cannula body 114 may contain three sub-sections, a tapered fitting 108, a tapered midsection 130 and an aorta interface 132. These subsections can be seen in Figure 1 as well as in various side views of the cannula body 114 depicted in Figures 2a-2b. In one embodiment, the cannula body 114 is made from injection molded clear polycarbonate. However, one of skill in the art would understand that the cannula body can be made from other types of plastic or any other suitable material.

One of skill in the art would recognize that the while the shape of the cannula body 114 should be generally cylindrical, the opening need not be perfectly circular. The three sub-sections, tapered fitting 108, tapered midsection 130, and aorta interface 132, may be of different lengths relative to one another. In addition the different subsections may be made from one piece and they may have the same diameter. One of skill in the art would also recognize that the taper angle in the sub-sections, tapered fitting 108, tapered midsection 130, and aorta interface 132, may vary so long as the aorta interface reaches a diameter within the typical range of the diameter of an human aorta.

One end of the aortic cannula 100 forms tapered fitting 108. The tapered fitting is sized to couple to a female connector on an organ chamber (not shown) to create a seal. A threaded locking nut 102, pictured in Figure 1, is used to reversibly secure the aortic cannula 100 to the organ chamber (not shown). In one embodiment, the locking nut 102 has four wings 104 extending from its outer surface that are used for gripping and turning the locking nut 102. In one embodiment the wings 104 are rectangular. One of skill in the art would understand that the wings 104 could be any shape or omitted. The locking nut 102 may have a lip protruding inward from its bottom edge that snaps over locking ridge 110 and into the locking groove 112 on the cannula body 114. The locking groove 112 and the locking ridge 110 can be seen in Figure 1 and Figures 2a-2b. Alternatively, the locking nut 102 may be secured to the cannula body 114 using other mechanisms known to one skilled in the art. Once the locking nut 102 is seated in the locking groove 112, the aortic cannula 100 is securely fastened to the organ chamber (not shown) by turning the locking nut 102. Perfusate can be perfused through the cannula into the heart without leaking. One of skill in the art would understand that other designs can be used to attach the aortic cannula 100 to the organ chamber to prevent leakage.

One of skill in the art would understand that the aortic cannula 100 can be connected to an organ care system or any other tube, device, or path of flow. In addition, one of skill in the art would appreciate that the locking nut 102 may be omitted in embodiments where the male-female connection between the aortic cannula 100 and the organ care system (not shown) is tight enough to prevent leakage. One of skill in the art would also recognize that the locking nut 102 could be replaced with other types of connectors generally used in the art to create a flow path between two tubes.

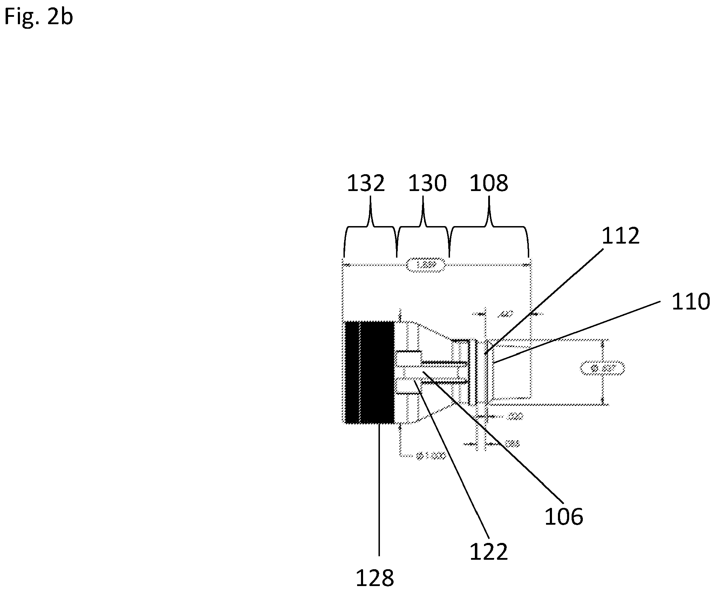

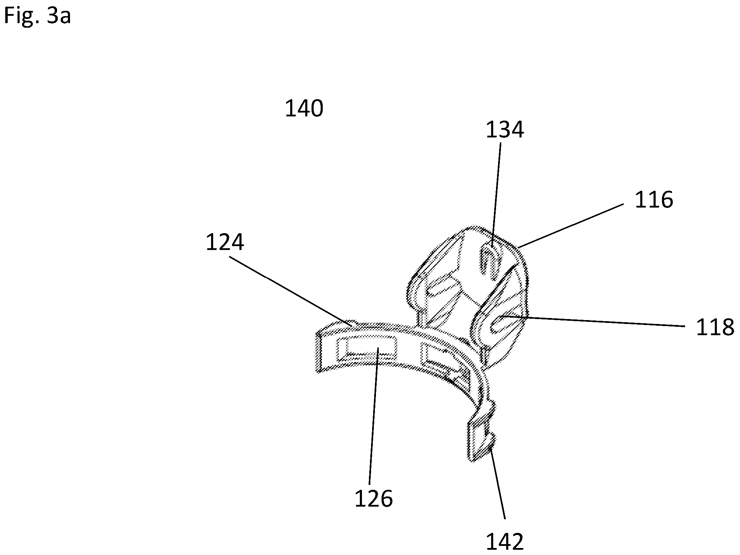

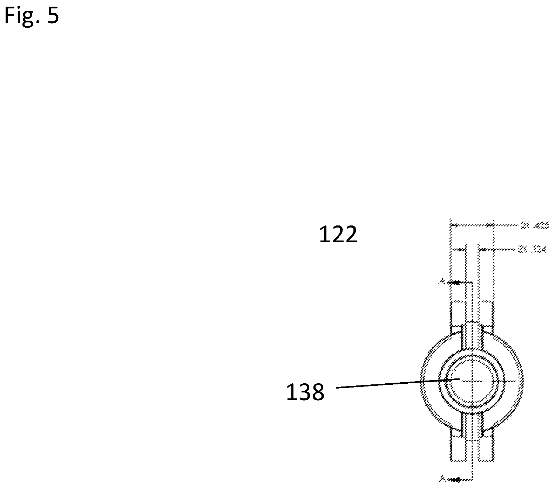

The tapered midsection 130 extends from the bottom edge of the tapered fitting 108 to the top edge of the aorta interface 132. The tapered midsection 130 reaches a final diameter the size of the aorta interface 132. The tapered midsection 130 helps to ensure smooth fluid flow from the aorta interface 132 to the tapered fitting 108. The tapered midsection 130 also helps minimize air trap and hemolysis and improve hemodynamics due to the smooth transition in flow path. The tapered midsection 130 has a pivot mount 122 and a spring pocket 106. The pivot mount 122 and the spring pocket 106 may be integrated with the tapered midsection 130. In one embodiment, the tapered midsection 130 has two pivot mounts 122 and two spring pockets 106, shown in Figures 1 and 2b. The pivot mounts 122 are located on each side of the cannula body 114. One of ordinary skill in the art would understand that one or more pivot mounts 122 and spring pockets 106 could be used. As shown in Figure 5, in one embodiment the pivot mount 122 has a circular center hole 138 sized to receive a dowel pin 120. The spring pocket 106 is located on the cannula body 114 and provides a space for a torsional spring (not shown). The dowel pin 120 fits through one side of the center hole 138 on the integrated pivot mount 122, through the center of the torsional spring in the spring pocket 106, and through the other side of the center hole 138 on the integrated pivot mount 122 . The torsional spring is oriented in spring pocket 106 such that depressing the pivot arm compresses the spring. One end of the torsional spring rests in the spring end pocket 134 on the thumb pad 116 seen in Figure 3a. One of ordinary skill in the art would understand that there are various ways to attach the pivot mount 122 to the cannula body 114 that allows the pivot mount 122 to pivot or move so that the aorta can be fit onto the cannula body 114 in operation. In one embodiment, the pivot mount 122 is made from injection molded polycarbonate, acetyl, or any suitable material.

One of skill in the art would also recognize that the torsional spring could be replaced with other types of spring loading mechanisms or omitted completely. The torsional spring could also be replaced by a molded leaf spring on the pivot arm or on the grip pad. With the use of a molded leaf spring the dowel pin would be omitted and cylindrical bosses on the cannula body 114 or a similar structure could be used to perform the same function.

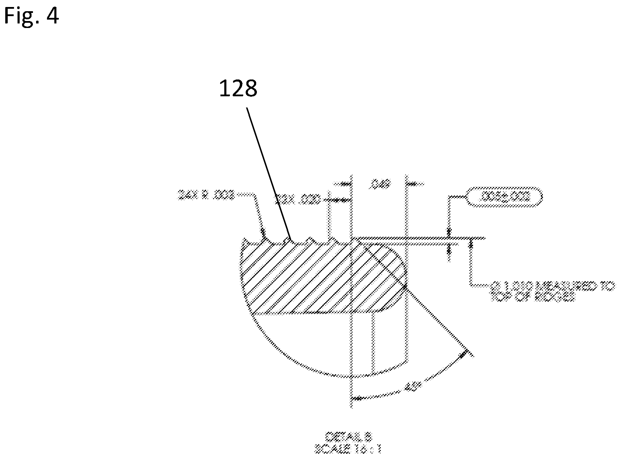

The aorta interface 132 is located adjacent the tapered midsection 130. The aorta interface 132 may be of a constant diameter and sized to fit within the aorta. The diameter of the aorta interface 132 can be between 1.27 and 5.08 cm (0.5 and 2 inches). In some embodiments the diameter of the aorta interface 132 can be between 1.9 and 2.858 cm (0.75 and 1.125 inches). Preferably, in some embodiments the diameter of the aorta interface is 1.905cm (0.75 inches), 2.22 cm (0.875 inches), 2.54 cm (1 inch), or 2.858 cm (1.125 inches). The aorta interface 132 may be smooth or textured. Figure 1 illustrates a texture 128 on the aorta interface 132 to help prevent the aorta from slipping off of the cannula body 114. In the embodiment shown in Figure 1, the aortic cannula 100 is placed in the aorta so that the aorta does not rise above the end of the texture 128. Figure 4 is a cross sectional view of one embodiment of the texture 128. The texture 128 may be of any shape. In one embodiment the texture 128 comprises concentric ridges extending around the aorta interface 132 that are sloped at a 45 degree angle on their lower side and are perpendicular to the cannula body 114 on their upper face. This design allows the aorta to slide onto the aorta interface 132 easily, but prevents the aorta from sliding off the aorta interface 132. Preferably the ridges are about 0.0127 cm (0.005 inches) tall. However, one of skill in the art would understand that the texture features could be of any shape and size to allow the aorta to be situated around the aorta interface 132 and to help hold the aorta in place while minimizing damage to the tissue. In one embodiment, the radial edge of the aortic interface 132 does not have a ridge to minimize trauma to the tissue. Alternatively, one of skill in the art would recognize that a ridge could be designed to minimize tissue trauma and to hold the aorta in place.

### Pivot Arm

A pivot arm 140 is coupled to the pivot mount 122. Figures 3a-e illustrate different views of a pivot arm and pivot arm strap (discussed below) in one embodiment. The pivot arm 140 allows the device 100 to adjust and grip aortas of different thicknesses. In one embodiment the cannula body 114 includes two pivot arms 140 coupled to two pivot mounts 122 on the cannula body. One of ordinary skill would understand that the number of pivot arms 140 corresponds to the number of pivot mounts 122. The pivot arm 140 comprises a grip pad 116, a sliding pivot window 118, and a strap 124. The sliding pivot window 118 allows the strap 124 to maintain uniform contact with the aorta through a range of motion. The grip pad 116 can be smooth, or contain features such as molded ridges or other texture to stop the user's fingers from slipping. The grip pad can be any shape, preferably round. In some embodiments the grip pad 116 may be detachable. In other embodiments a reusable tool that attaches to the pivot arms 140 could be used in place of the grip pads 116. The dowel pin 120 allows the pivot arm 140 to rotate around the dowel pin 120 when it is actuated. The pivot arm 140 is made from injection molded acetyl or any material with similar properties. One of skill in the art would recognize that while the sliding pivot provides certain advantages over a fixed pivot point, a fixed pivot point could also be used in embodiments outside the scope of the claims. Some embodiments may include a locking mechanism to hold the pivot arm 140 in an open position.

### Pivot Arm Strap

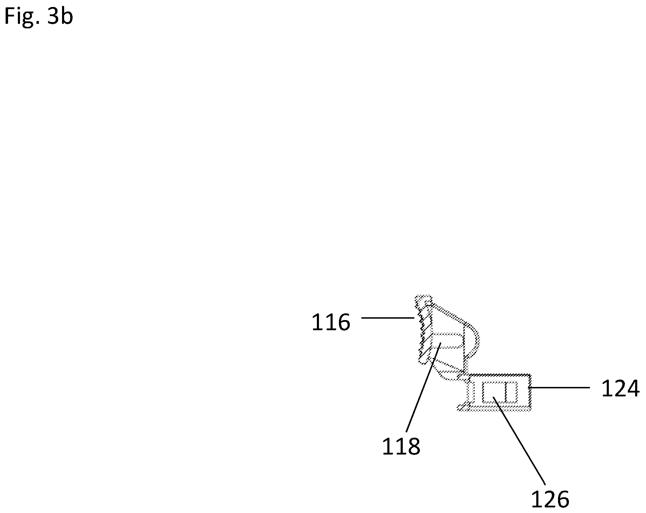

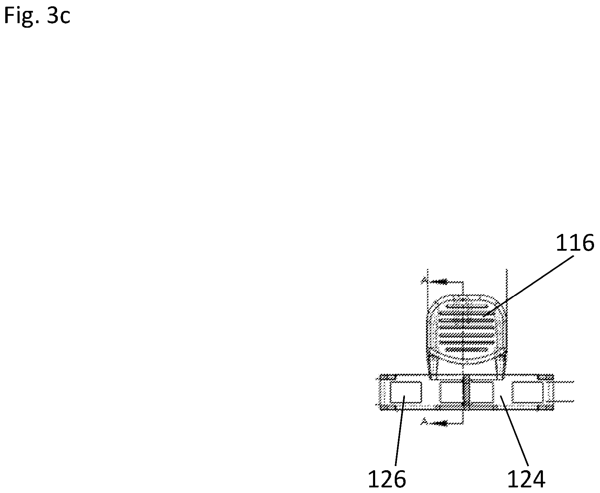

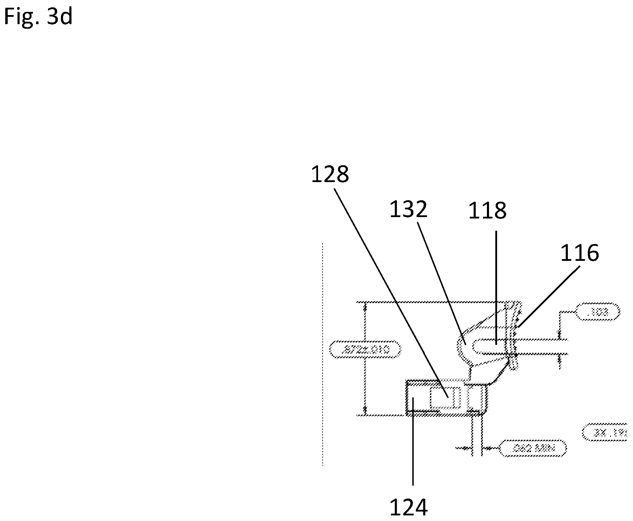

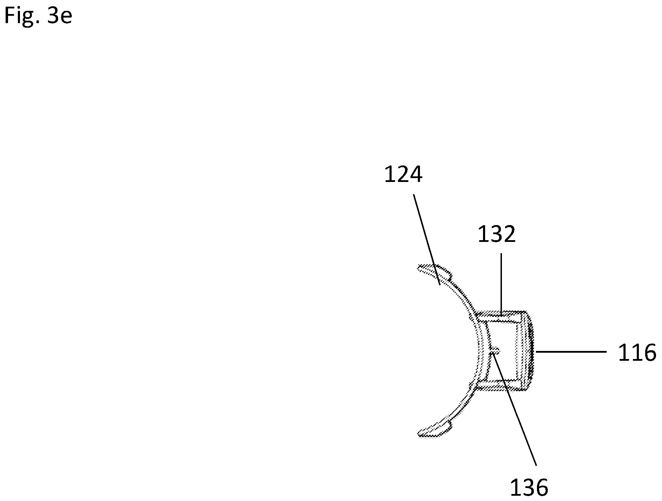

The pivot arm strap 124 is coupled to the pivot arm 140. The pivot arm strap is best seen in Figs. 1 and 3. As shown in Figure 1, in one embodiment the cannula body 114 includes two pivot arm straps 124 coupled to two pivot arms 140. One of ordinary skill would understand that the number of pivot straps 124 corresponds to the number of pivot arms 140. The pivot arm strap 124 and the sliding pivot window 118 allow the cannula body 114 to uniformly grip the aorta. The pivot arm strap 124 is designed to be stiff enough to hold the aorta, while maintaining enough flexibility to conform to the aorta and minimize tissue damage. The pivot arm straps 124 are curved. The pivot arm strap 124 optionally has a loop 136 and a guide 142 to retain a cable tie (not shown) around the pivot arm strap 124. The cable tie is made from a flexible nylon material or material with similar properties. Once the cable tie has been threaded through the loop 136 and slotted in the guide 142, it is tightened to the desired tension. The amount that the cable tie is tightened is the same for all sizes of cannulas. Windows 126 in the pivot arm strap 124 normalize the pressure exerted on the aorta by altering the surface area of the strap in contact with the aorta. Accordingly, the size of the windows 126 vary depending on the size of the aorta. The size of the windows 126 are calculated so that when the cable tie is tightened, it exerts the same compression on the aorta for every size device 100. Thus, the compression exerted on the aorta holds it in place without damaging the tissue. One of ordinary skill would understand that alternatively, the cable tie may be tightened to a specific tension for each size of the device 100. In addition, other mechanisms of clamping to hold the aorta in place could be used in place of the cable tie, for example a hose clamp or a tension strap. Additionally, the pivot arm strap 124 and the windows 126 could be of different shapes and sizes. Alternatively, the windows could be omitted. One of skill in the art would also understand that the pivot arm 140 and the pivot arm strap 124 could be sections of a single piece. In addition, one of skill in the art would understand that the inner surface of the pivot arm strap 124 could be smooth, or textured for additional traction.

In use, the aorta is secured to the cannula body. The grip pad 116 is depressed by the user causing the pivot arm 140 to move around the sliding pivot window 118 and to compress torsional spring. The pivot arm 140 rotates around the dowel pin 120 in the sliding pivot window 118 and the pivot arm straps 124 move away from the cannula body 114, which makes room to place the cannula in the aorta in a preferred manner than if the pivot point were fixed. When the grip pad 116 is released the torsional spring (not shown) exerts pressure on the pivot arm strap 124 and temporarily holds the aorta in place. The straps closes on the aorta and the sliding pivot window 118 allows the pivot point to change in order to compensate for variations in tissue thickness and maintain alignment and concentricity of pivot arm 140 to cannula body 114 through the full range of rotation. This allows the strap 124 to seat uniformly on the aorta. Then, the cable tie is threaded through the loop 136 and between the guide 142. The cable tie is tightened to a predetermined tension. One of skill in the art would understand that the cable tie could be replaced with other mechanisms for securing the pivot arm straps 124. In some embodiments the cable tie can come preassembled in the loops 136.

### Pledgets

The user may suture surgical felt pledgets on the aorta. The pledgets serve as an additional measure to retain the aorta on the cannula body 114 because the pledgets provide a barrier that does not slide between the pivot arm strap 124 and the cannula body 114. Four sets of two (one inside, one outside) pledgets are equally spaced around the aorta and sutured. One of skill in the art will recognize that more or fewer pledgets may be used. In one embodiment, the aorta is positioned onto the cannula body 114 so that the pledgets are not directly above a space between the pivot arms 140 to prevent the pledgets from sliding through the space between the two sides of the pivot arm straps 124. It will be recognized by one of skill in the art that the pledgets may be placed anywhere on the aorta and end up in any orientation with respect to the pivot arm straps. The pledgets may be standard, surgical felt pledgets. Alternatively, they may be injected molded, rigid, elastomeric pledgets made of a high Durometer material, such as silicone, or a similar material. One of skill in the art would understand that the pledgets could be replaced with other materials that attach to the tissue, and that provide an anchor to prevent the device from sliding between the strap and the cannula body or damaging the tissue. Examples of these materials include, but are not limited to, a continuous ring of material that attaches to the tissue or a staple.

### Tip Holder

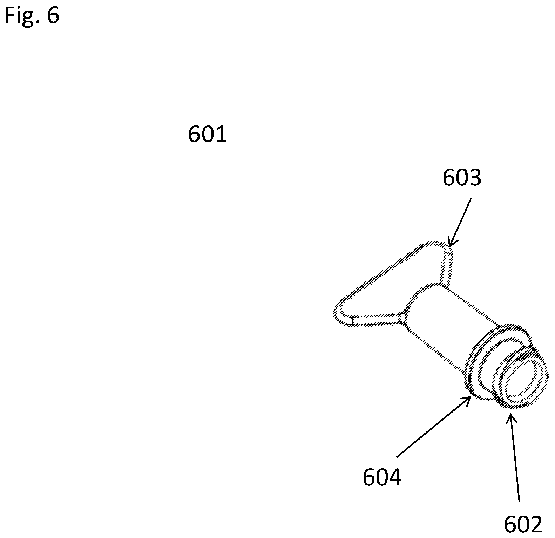

Fig. 6 depicts a tip holder 601. The tip holder 601 is generally cylindrical, though it may have other shapes. The tip holder has a handle 603. The handle may take any shape that allows a user to hold the tip holder 601. The tip holder 601 can also have threads 602. The locking nut 102 can be screwed onto the threads 602. The tip holder 601 can also have a stopper 604 which protrudes from the tip holder 601 and serves as a stopping point for the locking nut 102. One of skill in the art would understand that other designs can be used to attach the locking nut to the tip holder. Alternatively, the tip holder may be secured to the aortic cannula 100 using other mechanisms known to one skilled in the art. Once secured, the tip holder can be used to hold the aortic cannula 100 with or without a heart positioned on the aortic cannula 100.

### Example 1

The aortic cannula 100 may be used to connect a heart to an organ chamber (not shown). The aortic cannula 100 holds the aorta open and in place and allows perfusate to be perfused through the heart so the heart can be maintained in near physiologic conditions. To deploy the aortic cannula, the user first selects an aortic cannula 100 that is sized to fit the heart. In one embodiment the aortic cannula 100 may be selected by measuring the aorta. The user depresses the thumb pads 116 on the spring-loaded pivot arms. When the user depresses the grip pads 116, the pivot arms 140 rotate around the dowel pin 120 within the sliding pivot window 118 and the pivot arm straps 124 move away from the cannula body 114 making room to place the cannula in the aorta. The user can place the cannula in the aorta. Then the user releases the thumb pads allowing the pivot arms 140 to close on the aorta. The pivot arms 140 may be operated at the same time or individually. The pressure created by the torsional springs temporarily holds the aorta in place. The user may adjust the aorta position, if necessary, such that aorta is fully engaged on the cannula body 114. Next the user places a cable tie through the loops 136 and guides 142 in the pivot arm straps 124. The user then tightens the cable tie to hold the aorta in place. In some embodiments the cable tie may be tightened using a tool which tightens the cable tie to a predetermined force. The user inserts the tapered fitting 108 into the organ chamber (not shown). Then the user tightens the locking nut 102. One of skill in the art will recognize that in some embodiments the aortic cannula 100 could first be seated in the organ chamber and then the aorta could be secured to the aortic cannula 100.

## Claims

1. An aortic cannula for use in an ex-vivo organ care
system, the aortic cannula comprising:
a cannula body (114) comprising:
a fitting (108) configured to connect to an ex-vivo organ care system; and
an aorta interface (132) configured to contact an inner surface of an aorta of an ex-vivo heart; and
a pivot arm (140) that is coupled to a pivot mount (122), the pivot arm comprising:
a pivot arm strap, wherein the pivot arm strap (124) is operably connected to the pivot mount; and
a sliding pivot window (118) configured to allow the pivot arm strap to maintain uniform contact with the aorta through a range of motion,
wherein the pivot arm (140) is configured to allow the pivot arm strap (124) to uniformly contact an outer surface of the aorta to hold the aorta on the aorta interface (132) after the inner surface of the aorta is in contact with the aorta interface (132).

2. The aortic cannula of claim 1, wherein the cannula body (114) is comprised of injectionmolded polycarbonate.

3. . The aortic cannula of claim 1, wherein the cannula body (114) is substantially cylindrical.

4. . The aortic cannula of claim 1, wherein the fitting is a tapered fitting (108) and the cannula body further comprises a tapered midsection (130).

5. . The aortic cannula of claim 4, wherein the tapered midsection (130) extends from a bottom edge of the tapered fitting (108) to a top edge of the aorta interface (132), and wherein the tapered midsection has a larger diameter at the top edge compared to the bottom edge.

6. . The aortic cannula of claim 1, wherein the aorta interface (132) has a diameter between about 1.27 cm (0.5 inches) and about 5.1 cm (2 inches).

7. . The aortic cannula of claim 1, comprising a connector configured to reversibly secure the aortic cannula to an organ chamber of the ex-vivo organ care system.

8. . The aortic cannula of claim 7, wherein the connector is a threaded locking nut (102), wherein the threaded locking nut comprises an edge and a lip protruding inward from the edge, wherein the cannula body (114) comprises a locking ridge and a locking groove, and wherein the lip is configured to snap over the locking ridge (110) on the cannula body and into the locking groove (112) on the cannula body.

9. . The aortic cannula of claim 8, wherein the threaded locking nut (102) comprises an outer surface and four wings (104) extending from the outer surface, wherein the wings are configured to allow gripping and turning of the threaded locking nut.

10. . The aortic cannula of claim 1, wherein the pivot arm (120) comprises a grip pad (116).

11. . The aortic cannula of claim 1, wherein the pivot arm comprises a dowel pin (120), wherein the dowel pin is configured to allow the pivot arm to rotate around the dowel pin (120), and wherein the pivot mount comprises a circular center hole configured to receive the dowel pin of the pivot arm.

12. . The aortic cannula of claim 1, wherein the pivot arm (140) comprises a molded leaf spring.

13. . The aortic cannula of claim 4, wherein the tapered midsection (130) comprises the pivot mount (122) and a spring pocket (106) configured to receive a torsional spring.

14. . An organ care system comprising the aortic cannula of any of claims 1-13.

## Patentansprüche

1. Aortenkanüle zur Verwendung in einem Ex-vivo-Organpflegesystem, wobei die Aortenkanüle Folgendes umfasst:
einen Kanülenkörper (114), umfassend:
ein Anschlussstück (108), das dazu ausgebildet ist, an ein Ex-vivo-Organpflegesystem angeschlossen zu werden; und
eine Aortenschnittstelle (132), die dazu ausgebildet ist, eine Innenfläche einer Aorta eines Ex-vivo-Herzens zu berühren; und
einen Schwenkarm (140), der mit einer Schwenkhalterung (122) gekoppelt ist, wobei der Schwenkarm Folgendes umfasst:
einen Schwenkarmgurt, wobei der Schwenkarmgurt (124) mit der Schwenkhalterung wirkverbunden ist; und
ein verschiebbares Schwenkfenster (118), das dazu ausgebildet ist, es dem Schwenkarmgurt zu ermöglichen, über einen Bewegungsbereich hinweg einen gleichmäßigen Kontakt mit der Aorta aufrechtzuerhalten,
wobei der Schwenkarm (140) dazu ausgebildet ist, es dem Schwenkarmgurt (124) zu ermöglichen, eine Außenfläche der Aorta gleichmäßig zu berühren, um die Aorta an der Aortenschnittstelle (132) zu halten, nachdem die Innenfläche der Aorta mit der Aortenschnittstelle (132) in Kontakt gekommen ist.

2. Aortenkanüle nach Anspruch 1, wobei der Kanülenkörper (114) aus spritzgegossenem Polycarbonat besteht.

3. Aortenkanüle nach Anspruch 1, wobei der Kanülenkörper (114) im Wesentlichen zylindrisch ist.

4. Aortenkanüle nach Anspruch 1, wobei das Anschlussstück ein konisches Anschlussstück (108) ist und der Kanülenkörper ferner einen konischen Mittelabschnitt (130) umfasst.

5. Aortenkanüle nach Anspruch 4, wobei sich der konische Mittelabschnitt (130) von einer Unterkante des konischen Anschlussstücks (108) zu einer Oberkante der Aortenschnittstelle (132) erstreckt und wobei der konische Mittelabschnitt an der Oberkante einen größeren Durchmesser aufweist als an der Unterkante.

6. Aortenkanüle nach Anspruch 1, wobei die Aortenschnittstelle (132) einen Durchmesser zwischen etwa 1,27 cm (0,5 Zoll) und etwa 5,1 cm (2 Zoll) aufweist.

7. Aortenkanüle nach Anspruch 1, umfassend einen Verbinder, der dazu ausgebildet ist, die Aortenkanüle reversibel an einer Organkammer des Ex-vivo-Organpflegesystems zu befestigen.

8. Aortenkanüle nach Anspruch 7, wobei der Verbinder eine Gewindesicherungsmutter (102) ist, wobei die Gewindesicherungsmutter eine Kante und eine von der Kante nach innen ragende Lippe umfasst, wobei der Kanülenkörper (114) einen Verriegelungssteg und eine Verriegelungsnut umfasst und wobei die Lippe dazu ausgebildet ist, über den Verriegelungssteg (110) am Kanülenkörper und in die Verriegelungsnut (112) am Kanülenkörper zu schnappen.

9. Aortenkanüle nach Anspruch 8, wobei die Gewindesicherungsmutter (102) eine Außenfläche und vier sich von der Außenfläche erstreckende Flügel (104) umfasst, wobei die Flügel dazu ausgebildet sind, ein Greifen und Drehen der Gewindesicherungsmutter zu ermöglichen.

10. Aortenkanüle nach Anspruch 1, wobei der Schwenkarm (120) ein Griffpolster (116) umfasst.

11. Aortenkanüle nach Anspruch 1, wobei der Schwenkarm einen Passstift (120) umfasst, wobei der Passstift dazu ausgebildet ist, es dem Schwenkarm zu ermöglichen, sich um den Passstift (120) zu drehen, und wobei die Schwenkhalterung ein kreisförmiges Mittelloch umfasst, das dazu ausgebildet ist, den Passstift des Schwenkarms aufzunehmen.

12. Aortenkanüle nach Anspruch 1, wobei der Schwenkarm (140) eine geformte Blattfeder umfasst.

13. Aortenkanüle nach Anspruch 4, wobei der konische Mittelabschnitt (130) die Schwenkhalterung (122) und eine Federtasche (106) umfasst, die dazu ausgebildet ist, eine Torsionsfeder aufzunehmen.

14. Organpflegesystem, umfassend die Aortenkanüle nach einem der Ansprüche 1-13.

## Revendications

1. Canule aortique destinée à être utilisée dans un système de soins d'organes ex vivo, la canule aortique comprenant :
un corps de canule (114) comprenant :
un raccord (108) configuré pour être relié à un système de soins d'organes ex vivo ; et
une interface d'aorte (132) configurée pour entrer en contact avec une surface interne de l'aorte d'un cœur ex vivo ; et
un bras pivotant (140) qui est accouplé à un support de pivot (122), le bras pivotant comprenant :
une attache de bras pivotant, dans laquelle l'attache de bras pivotant (124) est reliée de manière fonctionnelle au support de pivot ; et
une fenêtre de pivotement coulissante (118) configurée pour permettre à l'attache de bras pivotant de maintenir un contact uniforme avec l'aorte sur toute une plage de mouvement,
dans laquelle le bras pivotant (140) est configuré pour permettre à l'attache de bras pivotant (124) d'entrer uniformément en contact avec une surface externe de l'aorte pour maintenir l'aorte sur l'interface d'aorte (132) après la mise en contact de la surface interne de l'aorte avec l'interface d'aorte (132).

2. Canule aortique selon la revendication 1, dans laquelle le corps de canule (114) est composé de polycarbonate moulé par injection.

3. Canule aortique selon la revendication 1, dans laquelle le corps de canule (114) est sensiblement cylindrique.

4. Canule aortique selon la revendication 1, dans laquelle le raccord est un raccord conique (108) et le corps de canule comprend en outre une section médiane conique (130).

5. Canule aortique selon la revendication 4, dans laquelle la section médiane conique (130) s'étend d'un bord inférieur du raccord conique (108) à un bord supérieur de l'interface d'aorte (132), et dans laquelle la section médiane conique a un diamètre plus grand au bord supérieur qu'au bord inférieur.

6. Canule aortique selon la revendication 1, dans laquelle l'interface d'aorte (132) a un diamètre compris entre environ 1,27 cm (0,5 pouce) et environ 5,1 cm (2 pouces).

7. Canule aortique selon la revendication 1, comprenant un connecteur configuré pour fixer de manière réversible la canule aortique à une chambre d'organe du système de soins d'organes ex vivo.

8. Canule aortique selon la revendication 7, dans laquelle le connecteur est un écrou de verrouillage fileté (102), dans laquelle l'écrou de verrouillage fileté comprend un bord et une lèvre faisant saillie vers l'intérieur à partir du bord, dans laquelle le corps de canule (114) comprend une crête de verrouillage et une rainure de verrouillage, et dans laquelle la lèvre est configurée pour s'enclencher sur la crête de verrouillage (110) du corps de canule et à l'intérieur de la rainure de verrouillage (112) sur le corps de canule.

9. Canule aortique selon la revendication 8, dans laquelle l'écrou de verrouillage fileté (102) comprend une surface externe et quatre ailes (104) s'étendant à partir de la surface externe, dans laquelle les ailes sont configurées pour permettre la préhension et la rotation de l'écrou de verrouillage fileté.

10. Canule aortique selon la revendication 1, dans laquelle le bras pivotant (120) comprend un tampon de préhension (116).

11. Canule aortique selon la revendication 1, dans laquelle le bras pivotant comprend une tige de positionnement (120), dans laquelle la tige de positionnement est configurée pour permettre au bras pivotant de tourner autour de la tige de positionnement (120), et dans laquelle le support de pivot comprend un trou central circulaire configuré pour recevoir la tige de positionnement du bras pivotant.

12. Canule aortique selon la revendication 1, dans laquelle le bras pivotant (140) comprend un ressort à lames moulé.

13. Canule aortique selon la revendication 4, dans laquelle la section médiane conique (130) comprend le support de pivot (122) et une poche à ressort (106) configurée pour recevoir un ressort de torsion.

14. Système de soins d'organes comprenant la canule aortique selon l'une quelconque des revendications 1 à 13.
